# FASCICULE DE BREVET EUROPEEN

(11) **EP 1 250 159 B1**
(45) Date de publication et mention de la délivrance du brevet: **30.03.2005**
(21) Numéro de dépôt: 01903989.0
(22) Date de dépôt: 29.01.2001
(51) Int. Cl.: A61L 2/02

(54) **STERILISATION PAR HAUTES PRESSIONS DE PRINCIPES ACTIFS SENSIBLES**
HOCHDRUCK-STERILISATION VON EMPFINDLICHEN WIRKSTOFFEN
HIGH PRESSURE STERILISING OF SENSITIVE ACTIVE PRINCIPLES

(30) Priorité: 27.01.2000 FR 0001059
(43) Date de publication de la demande: 23.10.2002
(73) Titulaire: Ellipse Pharmaceuticals, 33600 Pessac (FR)
(72) Inventeur: GRISLAIN, Luc, F-33290 Blanquefort (FR); VALLAYER, Bruno, F-33000 Bordeaux (FR); DEMAZEAU, Gérard, F-33170 Gradignan (FR); LARGETEAU, Alain, F-33610 Cestas (FR); LEMAGNEN, Gilles, F-33370 Tresses (FR); RIGALDIE, Yohan, F-33000 Bordeaux (FR)
(74) Mandataire: Ramey, Daniel
(86) Numéro de dépôt international: PCT/FR2001/000270
(87) Numéro de publication internationale: WO 2001/054737

(56) Documents cités:
- WO-A-00/72703
- WO-A-98/47503
- FR-A- 2 740 993

## Description

La présente invention concerne un procédé de stérilisation par des hautes pressions de principes actifs sensibles notamment des peptides, des oligonucléotides, ou des protéines.

On connaît la stérilisation de produits agro-alimentaires par la pression comme le procédé décrit dans la demande de brevet européen N°89 4440. Ce procédé permet de traiter des liquides en continu par traitement desdits liquides dans une série d'étages dans lesquels les pressions augmentent au fur et à mesure. Lorsque le liquide atteint le dernier étage à très haute pression, le liquide est soumis à une dépression rapide qui brise les micro-organismes.

D'autres procédés prévoient de combiner la très haute pression avec les hautes températures. C'est ainsi que les aliments faiblement acides, déjà conditionnés, sont soumis à une température de pré-pressurisation puis pendant un instant bref, l'ensemble est porté à une très haute pression, ce qui fait monter la température instantanément à des valeurs importantes. La pression est ramenée à la pression de départ si bien que la température redescend immédiatement.

Ces procédés permettent de détruire les micro-organismes tels que virus, bactéries et moisissures dans des liquides car les liquides transmettent parfaitement les pressions exercées, ceci de façon isostatique.

La demande de brevet allemand DE-A-19 905159 décrit un procédé pour la stérilisation de substances ou de principes actifs prévus pour être introduits dans des médicaments à des pressions comprises entre 20 et 9 000 bars et à des températures comprises entre 25°C et 200°C.

De telles températures provoquent la dégradation de certains principes actifs et le but est généralement la stérilisation de principes actifs très résistants.

La demande internationale de brevet WO 98 47503 divulgue un procédé pour stériliser des préparations à base d'ibuprofène. Le but est de provoquer une mise sous forte pression 3 000 à 10 000 bars, tout en restant à des températures inférieures à la température de fusion de l'ibuprofène.

Dans la demande de brevet FR-A- 2 740 993, il est mentionné une presse isostatique pour traiter des liquides chargés afin d'augmenter la durée de conservation, notamment dans l'agro-alimentaire.

Par contre, du point de vue économique, de tels procédés sont peu adaptés pour des produits à faible valeur ajoutée car ils engendrent des investissements importants qu'il est difficile d'amortir.

On connaît d'autres moyens de stérilisation par des procédés radioactifs mais dans ce cas, si certains rayonnements peuvent donner satisfaction, ils posent des problèmes complexes quant à leur manipulation et surtout soulèvent des questions quant aux risques pour l'environnement, liés à leur utilisation.

Les membranes stérilisantes sont aussi un bon moyen pour retenir les particules non souhaitées mais il faut aussi que le criblage convienne et que l'on puisse mécaniquement séparer les différentes catégories. De façon générale, les règles industrielles que la filtration impose, les rendent peu pratiques à mettre en oeuvre et les limitent aux seuls liquides.

La technique la plus courante consiste à stériliser par la chaleur sous pression comme indiqué ci-avant ou simplement par la chaleur, à la pression ambiante. Cette méthode n'est applicable qu'aux produits qui supportent la mise en température or de nombreux principes actifs sont sensibles à la chaleur. Les principes actifs, notamment issus des biotechnologies tels que les peptides ou les molécules issues des recherches sur l'ADN.

On connaît, par le document WO-A-00/72703, un procédé d'inactivation de micro-organismes dans un liquide alimentaire tel que du lait, qui consiste à soumettre ce liquide à un traitement d'homogénéisation par haute pression dynamique, dans lequel les micro-organismes sont détruits par une combinaison de mécanismes comprenant une chute brutale de pression, des contraintes de cisaillement, une cavitation et des chocs. Le procédé est applicable en continu à un produit liquide, mais non à des principes actifs sensibles qui peuvent ne pas être sous forme liquide:

Le procédé selon la présente invention propose des étapes permettant de stériliser, par un traitement sous hautes pressions, des principes actifs sensibles, sans les dégrader, et dans des conditions de mise en oeuvre adaptées à l'industrie. Un tel procédé, appliqué à des entités thérapeutiques à fortes valeurs ajoutées, est économiquement adapté mais il subsiste un préjugé qui veut que les principes actifs sensibles sont dégradés par le passage à de très hautes pressions et que l'utilisation de très hautes pressions comme moyen bactéricide et virucide n'est pas adapté.

Contrairement à ce qui est connu de l'art antérieur, la température, dans les plages négatives, permet de renforcer les effets de la stérilisation sous pression.

On note aussi que l'application de ce procédé de stérilisation à des solides sous forme divisée est particulièrement révélateur des capacités de stérilisation de produits autres que les liquides.

A cet effet, le procédé de stérilisation d'au moins un principe actif sensible selon l'invention, se caractérise en ce que ledit principe actif est un peptide, un anticorps, une protéine ou une enzyme et est soumis à un pressage isostatique à des pressions comprises entre 3000 et 6000 bars et à des températures comprises entre -30°c et +25°C

Plus particulièrement, le principe actif traité est d'un poids moléculaire inférieur ou égal à 170 000 g/mol.

Préférentiellement le procédé est conduit à des températures négatives jusqu'à -30°C et même directement sur une formulation contenant le principe actif en milieu pulvérulent.

Afin d'atteindre l'effet stérilisant recherché, on ajuste la pression et la température pour atteindre une réduction logarithmique de contamination des micro-organismes supérieure à 6.

Le procédé est maintenant décrit en donnant plusieurs exemples et résultats d'essais, en regard des dessins annexés sur lesquels les différentes figures représentent :
- figure 1A: courbe de cinétique de dissolution de l'ibuprofène en comprimé obtenu par pressage isostatique sous hautes pressions,
- figure 1 B, courbe de cinétique de dissolution comparée de l'ibuprofène en comprimé obtenu par pressage uni-axial sous hautes pressions,
- figures 2A-2D : courbes chromatographiques de l'insuline après traitement sous hautes pressions,
- figure 3 : courbe de l'activité immunologique des anticorps traités sous hautes pressions,
- figure 4 : tableau des résultats obtenus avec les tests sur *Pseudomonas aeruginosa*
- figure 5 : tableau des résultats obtenus avec les tests sur *Candida albicans*
- figure 6 : tableau des résultats obtenus avec les tests sur *Aspergillus niger* sporulé, et
- figure 7 : tableau des résultats obtenus avec les tests sur *Bacillus subtilis,* directement sur un milieu pulvérulent.

On définit tout d'abord les possibilités de traitement des molécules fragiles par les hautes pressions en montrant que la structure des principes actifs reste inchangée après soumission à de telles pressions.

On entend par éléments fragiles les molécules comme les protéines, les anticorps, les peptides, les enzymes, mais aussi les vecteurs médicamenteux tels que les microsphères, les nanocapsules, ou les liposomes. Ces éléments fragiles ne peuvent supporter des montées en température ou des rayonnements.

On sait que lors des pressages isostatiques, très peu d'énergie est transmise aux produits traités, ce qui préserve leur potentiel.

La description qui va suivre est applicable à la stérilisation de poudres, comme les poudres pour préparations extemporanées destinées à être injectées. De plus, il est possible de réaliser directement par pressage, des comprimés à partir de poudres, dans la forme voulue, et stérilisés pour être implantables. Il convient seulement de prévoir une matrice adaptée pour obtenir une forme et une masse voulue, de façon reproductible.

Les essais conduits l'ont été avec un anti-inflammatoire non stéroïdien du groupe des propioniques, dérivé de l'acide carboxylique, l'ibuprofène, sous forme de base.

Plus particulièrement, on retient la formule suivante :
- IBUPROFENE : 60% ( fabrication de la société Knoff sous le code produit UPSA)
- LACTOSE : 35% ( fabrication de la société Seppic sous le code produit A 16 M07)
- AEROSIL 200 : 0,5% ( fabrication de la société Degussa sous le code produit M01 03 E330)
- STEARATE DE Mg 200 : 0,5% ( fabrication de la société Cpf sous le code produit M01 O3 E677)
- TALC : 4,0% ( fabrication de la société Cpf sous le code produit M01 03 E044)

On réalise des comprimés par pressage isostatique à 2000 bars.

On effectue des tests de dissolution avec une agitation à 100 tours minutes, à 37°C et un pH de 7,2.

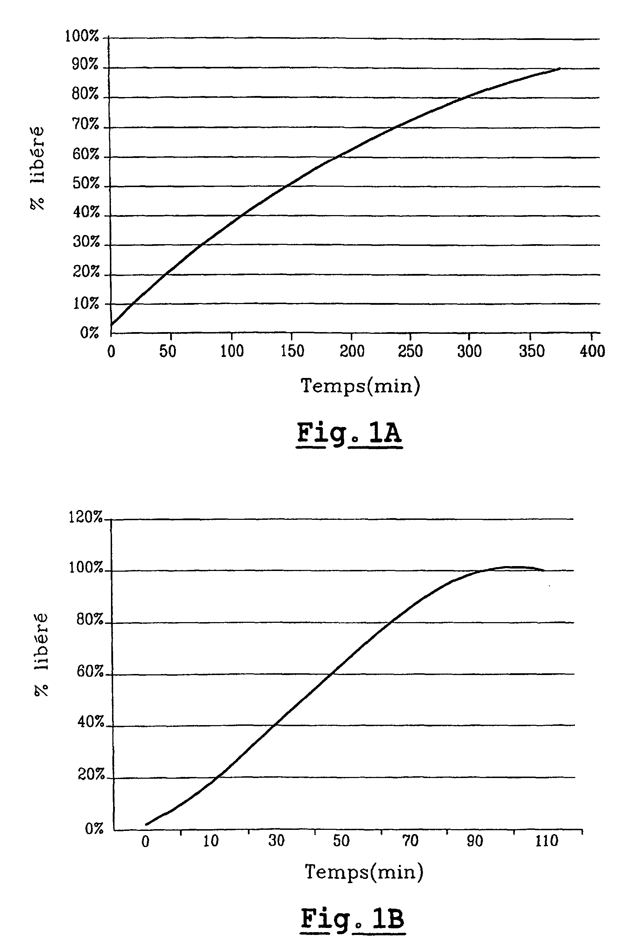

La courbe de la figure 1 A montre que la cinétique de dissolution dans le cas d'un pressage isostatique est pratiquement linéaire mais avec une durée allongée de libération du principe actif de près de 4 heures.

A titre de comparaison, on a réalisé une compression uni-axiale avec la même formulation et avec la même pression exercée, la courbe correspondante est indiquée sur la figure 1 B. On note que la totalité de l'ibuprofène est libérée en moins de deux heures.

Un premier avantage du pressage isostatique est une cinétique de dissolution améliorée.

Des essais complémentaires sont conduits pour montrer l'innocuité du traitement par les hautes pressions sur les éléments fragiles. En l'occurrence ces éléments sont mis en solution ou sous forme de poudre.

On a retenu à cet effet des éléments fragiles de poids moléculaire inférieur ou égal 170 000 g/mol tels que les peptides, les protéines et les anticorps et plus particulièrement :
- un décapeptide : gramicidine (PM=1 140 g/mol), sous forme de poudre,
- l'insuline 40 UI (PM = 5 800 g/mol), et
- un anticorps monoclonal, comme l'anticorps antivirus grippe A (PM = 150 000 g/mol).

a/ Le peptide, en l'occurrence la Gramicidine, est soumis à un traitement sous hautes pressions jusqu'à 5 000 bars, et sa pureté a été testée également par HPLC, les variations restent inférieures à 1 %. Les fortes pressions n'ont pas d'influence sur la structure.

b/ L'insuline a été choisie à 40 UI, du commerce, et diluée à 1/100 au pH 2,5. Pour la mesure, le passage est réalisé avec un débit de 1 ml/min dans un appareil d'HPLC (High Pressure Liquid Chromatographie).

Différents échantillons sont soumis, à température ambiante et à température négative -20°C à :
- la pression atmosphérique pour le témoin,
- 4 000 bars pendant 10 min,
- 5 000 bars pendant 10 min, et
- 6 000 bars pendant 10 min.

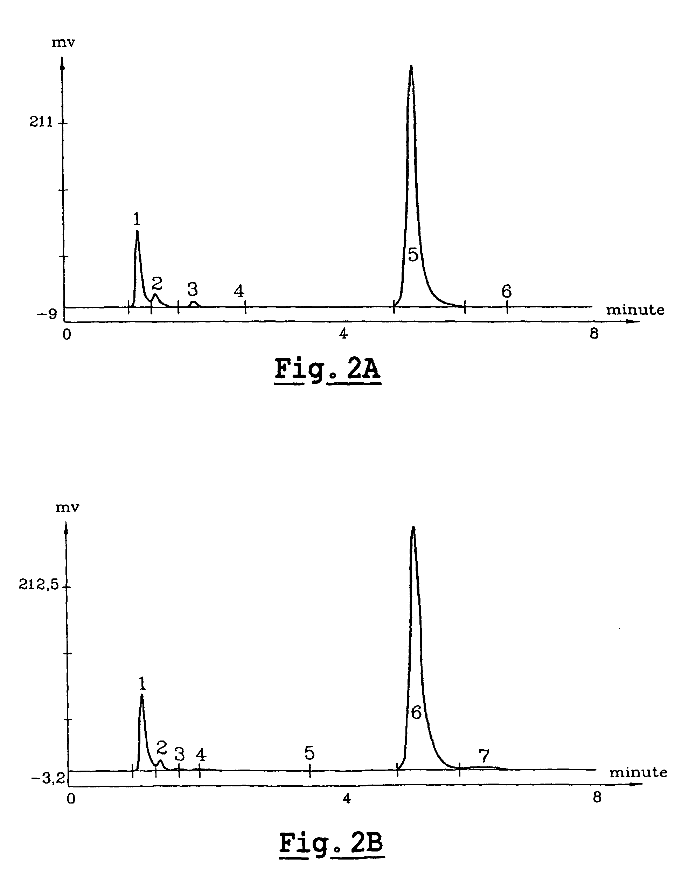

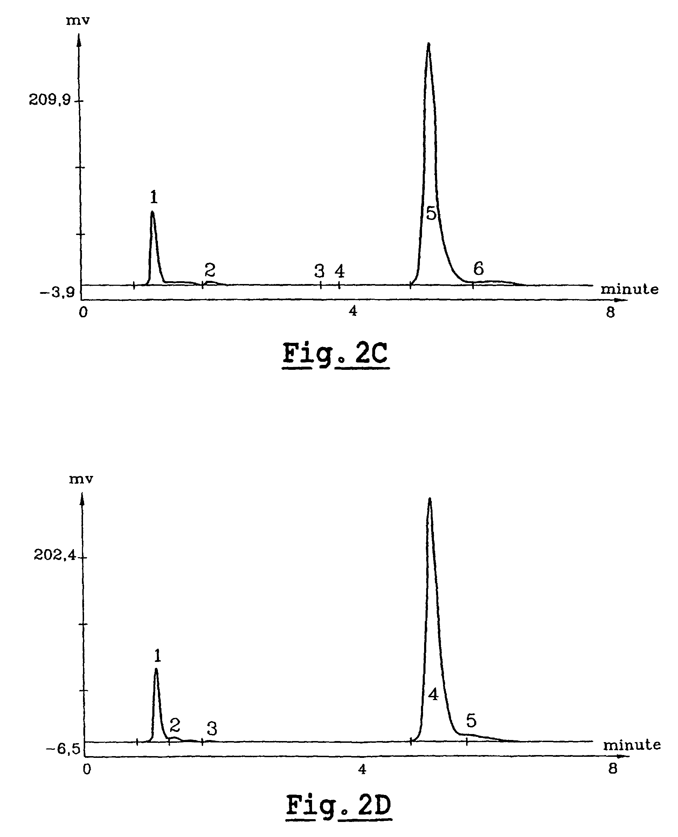

Les échantillons, après traitement, sont ensuite analysés à 214 nm dans un appareil de chromatographie avec une injection automatique. On obtient les résultats suivants montrés sur les figures 2A,2B et 2C et 2D.

On constate que dans les différentes courbes des échantillons soumis à une stérilisation par traitement sous hautes pressions, il apparaît les mêmes pics avec sensiblement les mêmes intensités et centrés sur les mêmes plages de temps que dans le cas du témoin à pression atmosphérique, y compris lorsque la température est négative.

Il n'y a pas de modifications ou d'altérations notables de la structure du produit.

c/ En ce qui concerne les anticorps monoclonaux, on a testé des anticorps monoclonaux dirigés contre le virus de la grippe A de poids moléculaire 150 000 g/mol, que l'on a placés sous hautes pressions à 4 000, 5 000 et 6 000 bars pendant 10 min à température ambiante.

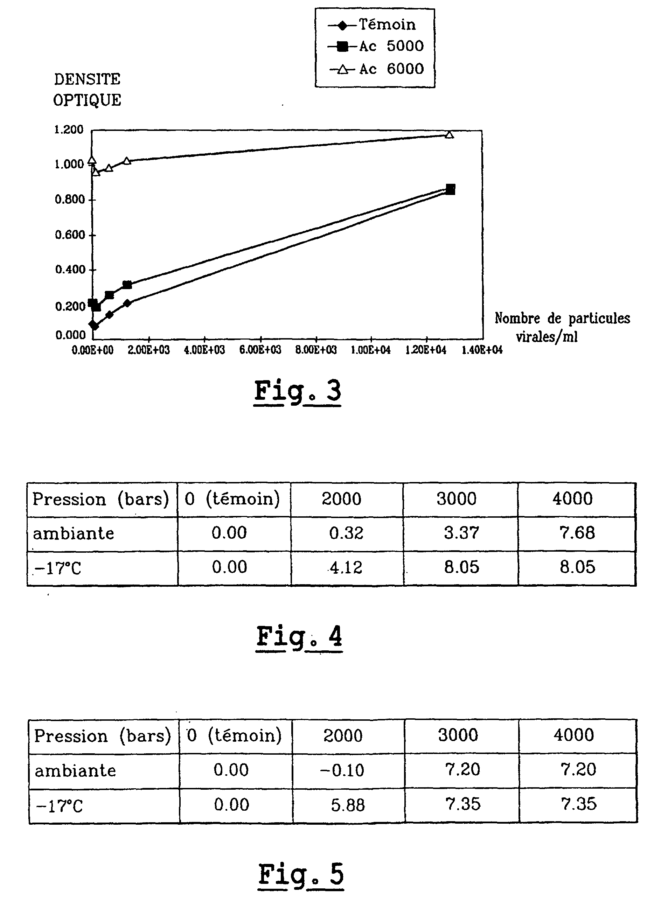

Un test ELISA montre que l'activité immunologique des anticorps traités jusqu'à 5 000 bars est conservée tandis que cette activité tend à diminuer au-delà de 6 000 bars. C'est ce que montre les courbes de la figure 3.

Puisque les hautes pressions ne dégradent pas les principes actifs, les essais suivants sont maintenant conduits pour montrer l'efficacité du traitement vis à vis des micro-organismes.

Comme il n'existe pas d'indicateur microbiologique d'efficacité normalisée pour qualifier les traitements stérilisants, on choisit quatre micro-organismes pathogènes pour constituer un échantillon des plus représentatifs, ces micro-organismes étant en milieu liquide :
- un gram - : *Pseudomonas aeruginosa*,
- une levure : *Candida albicans*, *et*
- une spore de moisissure : *Aspergillus niger* sporulé.

Selon la pharmacopée européenne, un traitement stérilisant exige une diminution de 6 log la population de micro-organismes.

### Tests sur : Pseudomonas aeruginosa

Les résultats sont regroupés dans le tableau de la figure 4 qui montre la réduction logarithmique. On constate que la pression produit toujours un effet stérilisant à condition d'atteindre des pressions importantes, 4 000 bars.

On note dans ces essais que des manipulations ont été conduites à température ambiante et en ambiance froide, -17°C. De façon surprenante, on constate une amélioration des effets stérilisants de la pression et dès 3 000 bars, l'effet stérilisant recherché est atteint.

### Tests sur Candida albicans

Ce micro-organisme est particulièrement sensible à la pression mais on constate de nouveau l'effet des basses températures qui renforcent l'action et permettent dès 2 000 bars d'atteindre pratiquement le différentiel de 6 log. Tableau de la figure 5.

### Tests sur Aspergillus niger sporulé

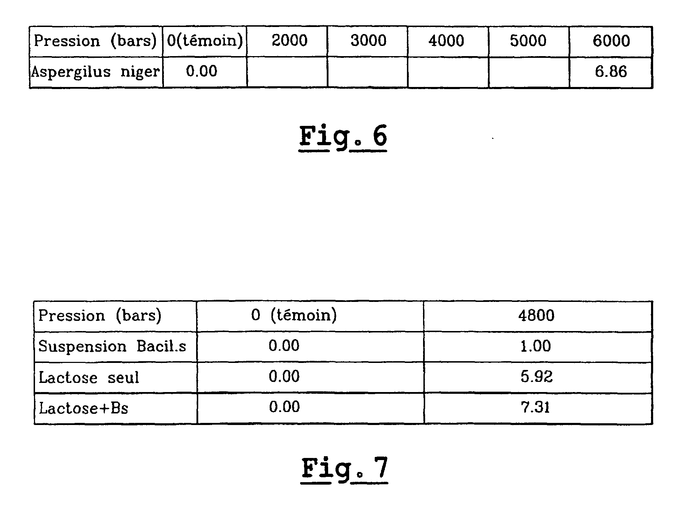

Les essais montrent qu'il faut atteindre 6 000 bars pour atteindre un taux de réduction logarithmique supérieur à 6, aidés par les effets associés engendrés par les températures froides à -17°C. Tableau de la figure 6.

D'autres essais sont conduits directement sur des milieux pulvérulents.

Le milieu pulvérulent retenu est le lactose, excipient que l'on retrouve dans la fabrication de nombreux compacts pharmaceutiques ou dans des poudres pour préparations injectables.

La souche implantée est *Bacillus subtilis.*

Afin de déterminer le bruit de fond du lactose qui pourrait perturber la mesure, on réalise un échantillon témoin de poudre. Ceci permet de tenir compte de la contamination éventuellement présente dans le lactose.

Chaque échantillon est placé dans une enveloppe étanche et soumis aux hautes pressions, 4 000 bars en l'occurrence, par pressage isostatique.

Les deux autres échantillons comprennent pour le premier, le lactose avec des bactéries *Bacillus subtilis*, sous forme lyophilisée et pour le second une même dose de bactéries *Bacillus subtilis,* lyophilisées mais simplement dans un diluant de façon à n'exposer que la charge bactérienne à l'action stérilisante.

Les résultats sont regroupés dans le tableau de la figure 7. Ils montrent que le lactose contenait déjà des micro-organismes et que ces résidants ont été détruits tout comme la charge ajoutée, la réduction logarithmique étant de 7,31.

On remarque une efficacité moindre du traitement par les hautes pressions sur la charge microbienne dans le milieu liquide.

On note aussi que les traitements sous pression avec une température négative permettent d'améliorer les performances de stérilisation en particulier lorsque la température est portée à -17°C voire -20°C et -30°C au lieu de la température ambiante pendant le traitement sous pression.

Aussi le traitement de stérilisation sous pression, entre 3 000 et 6 000 bars, notamment de poudres, selon la présente invention, peut être conduit dans une plage de températures couvrant -30°C à +25°C. Ce traitement n'affecte pas les principes actifs retenus

## Revendications

1. Procédé de stérilisation d'au moins un principe actif sensible, **caractérisé en ce que** ledit principe actif est un peptide, un anticorps, une protéine ou une enzyme et est soumis à un pressage isostatique à des pressions comprises entre 3 000 et 6 000 bars et à des températures comprises entre -30°C et +25°C.

2. Procédé de stérilisation selon la revendication 1, **caractérisé en ce que** le principe actif traité est d'un poids moléculaire inférieur ou égal à 170 000 g/mol.

3. Procédé de stérilisation selon la revendication 2, **caractérisé en ce que** le principe actif est de l'insuline.

4. Procédé de stérilisation selon l'une quelconque des revendications précédentes, **caractérisé en ce qu'**il est conduit à des températures négatives jusqu'à -30°C.

5. Procédé de stérilisation selon l'une quelconque des revendications précédentes, **caractérisé en ce que** le pressage isostatique est conduit directement sur une formulation contenant le principe actif en milieu pulvérulent.

6. Procédé de stérilisation selon l'une quelconque des revendications précédentes, **caractérisé en ce que** l'on ajuste la pression et la température pour atteindre une réduction logarithmique de contamination des micro-organismes supérieure à 6.

## Claims

1. Process of sterilization of at least one sensitive active principle, **characterized in that** the at least one active principle is a peptide, an antibody, a protein or an enzyme and is subjected to an isostatic pressure at pressures comprised between 3,000 and 6,000 bars and at temperatures comprised between -30°C and +25°C.

2. Process of sterilization according to claim 1, **characterized in that** the active principle treated has a molecular weight less than or equal to 170,000 g/mol.

3. Process of sterilization according to claim 2, **characterized in that** the active principle is insulin.

4. Process of sterilization according to any one of the preceding claims, **characterized in that** it is conducted at negative temperatures down to -30°C.

5. Process of sterilization according to any one of the preceding claims, **characterized in that** the isostatic pressure is conducted directly to a formulation containing the active principle in pulverulent medium.

6. Process of sterilization according to any one of the preceding claims, **characterized in that** the pressure and the temperature are adjusted to achieve a logarithmic reduction of contamination by microorganisms greater than 6.

## Patentansprüche

1. Sterilisationsverfahren für mindestens einen empfindlichen Wirkstoff, **dadurch gekennzeichnet, dass** der genannte Wirkstoff ein Peptid, ein Antikörper, ein Protein oder ein Enzym ist und einer isostatischen Druckanwendung mit Druckwerten zwischen 3000 und 6000 bar und Temperaturen zwischen -30 °C und +25 °C unterzogen wird.

2. Sterilisationsverfahren nach Anspruch 1, **dadurch gekennzeichnet, dass** der behandelte Wirkstoff ein Molekulargewicht unter oder gleich 170000 g/mol aufweist.

3. Sterilisationsverfahren nach Anspruch 2, **dadurch gekennzeichnet, dass** der Wirkstoff Insulin ist.

4. Sterilisationsverfahren nach einem der vorhergehenden Ansprüche, **dadurch gekennzeichnet, dass** es bei negativen Temperaturen bis -30 °C durchgeführt wird.

5. Sterilisationsverfahren nach einem der vorhergehenden Ansprüche, **dadurch gekennzeichnet, dass** die isostatische Druckanwendung direkt auf eine Formulierung durchgeführt wird, die den Wirkstoff in pulverförmigem Milieu enthält.

6. Sterilisationsverfahren nach einem der vorhergehenden Ansprüche, **dadurch gekennzeichnet, dass** der Druck und die Temperatur eingestellt werden, um eine logarithmische Reduzierung der Verunreinigung mit Mikroorganismen über 6 zu erreichen.
